# EUROPEAN PATENT APPLICATION

(11) **EP 2 312 317 A2**
(43) Date of publication of application: **20.04.2011**
(21) Application number: 10178669.7
(22) Date of filing: 03.05.2006
(51) Int. Cl.: G01N 33/58, G01N 33/542, G01N 21/64, C12Q 1/68

(54) **Fluorescent detection system and dye set for use therewith**

(30) Priority: 03.05.2005 US 677233 P
(62) Divisional of application: 06752160.9
(71) Applicant: Life Technologies Corporation, Carlsbad, CA 92008 (US)
(72) Inventor: Oldham, Mark, F., Los Gatos, CA 95033 (US); Boege, Steven, J., San Mateo, CA (US); King, Howard, Gregg, Berkeley, CA (US)
(74) Representative: Williams, Richard Andrew Norman

(57) **Abstract**

A system is provided that can comprise: at least two excitation sources, each adapted to provide a different excitation wavelength than at least one other; at least one detector; and a plurality of spectrally resolvable dyes. A set of dyes is also provided and can comprise one or more energy transfer dyes and each energy transfer dye can include two or more fluorescence resonance energy transfer dye moieties linked together. A set of energy transfer dyes is also provided wherein each energy transfer dye of the set comprises a different donor dye moiety than the other energy transfer dyes of the set, and the same acceptor dye moiety as the other energy transfer dyes of the set. A method of detection using the system is also provided.

## Description

### CROSS REFERENCE TO RELATED APPLICATION

The present application claims benefit from earlier filed U.S. Provisional Application No. 60/677,233, filed May 3, 2005, which is herein incorporated by reference in its entirety.

### FIELD

The present teachings relate to a detection system for detecting fluorescence from fluorescent dyes, and methods for detecting fluorescent dyes.

### BACKGROUND

For identification of biological and chemical sample compositions, various markers, for example, fluorescent dyes and energy transfer fluorescent dyes, can be added to a sample. Many fluorescent dyes have a large Stokes shift, that is, a large difference between the wavelength of maximum absorbance and the wavelength of maximum fluorescent emission. A large Stokes shift can enable a detection system to differentiation between the wavelength of fluorescent emission of a fluorescent dye and the wavelength emitted from an excitation source used to excite the fluorescent dye.

### SUMMARY

According to various embodiments, the present teachings provide for a detection system that comprises two or more excitation sources, at least one detector, and a set of dyes. The excitation sources can be adapted to emit a plurality of different individual excitation beam wavelength ranges, wherein each excitation source emits at least one wavelength that is not emitted in the excitation beam wavelength range of at least one other of the excitation sources. Each excitation source can comprise a respective individual radiation source or two or more excitation sources can comprise the same raditation source, For example, each excitation source can comprise a separate light-emitting diode (LED) or laser source, or two or more excitation sources can comprise a common broad spectrum light source and appropriate optics, filters, gratings, or the like. In an exemplary embodiment, a first excitation source can be provided that is adapted to emit a first excitation beam wavelength range of from about 460 nm to about 475 nm, and a second excitation source can be provided that is adapted to emit a second excitation beam wavelength range of from about 480 nm to about 495 nm. The second excitation beam wavelength range can be emitted at a different time or in a different direction than the first excitation beam wavelength range. In some embodiments, a group of excitation sources is provided that is adapted to emit two, three, four, or more, different and non-overlapping excitation beam wavelength ranges.

In some embodiments, the detection system can comprise a set of dyes and one or more of a sequence detection system, an electrophoretic detection system, a capillary electrophoresis system, a gel or slab electrophoresis system, a polymerase chain reaction (PCR) detection system, a real-time PCR detection system, a combination thereof, or the like,

According to various embodiments, a set of dyes is provided for use with a detection system as described herein, wherein at least two dyes of the set have different absorption wavelength ranges but the same or substantially overlapping emission wavelengths ranges. In some embodiments, the present teachings provide a set of dyes wherein at least one of the dyes of the set is an energy transfer dye. In some embodiments, at least one of the dyes of the set can comprise a donor dye moiety, an acceptor dye moiety, and optionally a linker moiety. In some embodiments, the present teachings provide a set of dyes comprising two or more energy transfer dyes wherein each energy transfer dye of the set of dyes includes a different donor dye moiety than the other energy transfer dyes in the set, and each energy transfer dye of the set of dyes includes an acceptor dye moiety that is of the same type or structure and/or has the same spectral properties and/or emission wavelength range as the acceptor dye moieties of the other energy transfer dyes of the set. In some embodiments, the present teachings provide a set of energy transfer dyes wherein each energy transfer dye of the set includes a different donor dye moiety than the other dyes in the set, and each energy transfer dye of the set includes an acceptor dye moiety that is of the same type or structure and/or has the same spectral properties and/or emission wavelength as the acceptor dye moieties of the other dyes of the set.

In some embodiments, a set of dyes is provided wherein at least one of the dyes comprises a donor dye moiety and an acceptor dye moiety that are covalently attached to one another by a linking moiety (also referred to herein as a linker or as a linker moiety). In some embodiments, the excitation energy emitted by the donor dye moiety can be absorbed by the acceptor dye moiety that can in-turn fluoresce. In some embodiments, the linker moiety can serve to facilitate the effective transfer of energy between the donor dye moiety and the acceptor dye moiety of at the least one energy transfer dye. In some embodiments, the energy transfer between a donor dye moiety and an acceptor dye moiety can be referred to as a fluorescence resonance energy transfer which is a distance-dependent interaction between the electronic excited states of the donor dye moiety and the acceptor dye moiety wherein excitation is transferred from the donor dye moiety to the acceptor dye moiety without emission of a photon from the donor dye moiety.

In some embodiments, by including different donor dye moieties in the respective energy transfer dyes, a series of energy transfer fluorescent dyes having spectrally resolvable excitation absorption can be provided, and by including the same type of acceptor dye moiety in each respective energy transfer dye of the set, the energy transfer dyes can emit within a common wavelength range while absorbing in different, spectrally resolvable wavelength ranges. As such, the set can be useful in conjunction with a detection system as described herein. In some embodiments, the detection system and a dye set of the present teachings can be useful in the detection of multiple target substances in a sample, such as in a nucleic acid sequence sequencing assay.

In some embodiments, the present teachings provide a set of dyes wherein at least one of the dyes of the set is an energy transfer dye and wherein two or more of the dyes of the set have emission wavelengths that substantially overlaps. In some embodiments, the present teachings provide a set of dyes wherein at least one of the dyes of the set is an energy transfer dye and wherein at least two subsets of the dye set comprise two or more of the dyes having emission wavelengths that substantially overlap. In some embodiments, at least one fluorescent dye that is not an energy transfer dye is included in a set of dyes that also include at least one energy transfer dye.

In some embodiments, the detection system can be used to detect fluorescence emitted from one or more sample-retainment regions, for example, from one or more containers, wherein each container is adapted to retain a respective sample or a respective aliquot or portion of a sample. In some embodiments, each sample-retainment region can include a sample disposed therein and each sample can include a set of dyes according to the present teachings. In some embodiments, each dye of the set and/or each donor dye moiety of each respective energy transfer dye of the set can absorb radiation at a different wavelength than the other dyes and/or other donor dye moieties of the set, and each dye of the set and/or each acceptor dye moiety of each respective energy transfer dye of the set can emit radiation at the same wavelength or at a wavelength range that substantially overlaps with at least one other dye and/or at least one other acceptor dye moiety of the set.

In some embodiments, a kit is provided that includes a set of dyes according to the present teachings. At least two dyes of the set can emit radiation at the same wavelength or within a substantially overlapping wavelength range as at least one other dye of the set.

In some embodiments, a method for detecting an analyte in a sample is provided that includes providing a sample having at least two detectable dyes, wherein each detectable dye emits radiation at the same wavelength, or within a substantially overlapping wavelength range, as that of another dye of the set, upon excitation by an appropriate radiation source that can excite the dyes. In some embodiments, the method can comprise the detection of dyes including at least one energy transfer dye. In some embodiments, the method comprises irradiating a sample with two or more different excitation beams, wherein each excitation beam includes a wavelength that falls within the peak absorption wavelength range of at least one respective dye of the set. The method can include detecting radiation that is emitted from the dyes as a result of their excitation. In some embodiments, the radiation that is emitted from the dyes can be of the same emission wavelength or within a substantially overlapping emission wavelength range. In some embodiments, the sample can be sequentially irradiated with two or more different excitation wavelength ranges.

### BRIEF DESCRIPTION OF THE DRAWINGS

Various embodiments of the present teachings are exemplified by the accompanying drawings. The teachings are not limited to the embodiments depicted, and include equivalent structures and methods as set forth in the following description and known to those of ordinary skill in the art. In the drawings:

Fig. 1 is a perspective view of a system according to various embodiments for detecting different analytes in a plurality of spaced-apart sample containers and including respective groups of four different excitation sources and one detector corresponding to each sample container;

Fig, 2 illustrates an exemplary system for nucleic acid sequencing using capillary electrophoresis and including a detection system according to various embodiments;

Fig. 3 is a graph showing the normalized fluorescence emission spectra of the four different fluorescent energy transfer dyes shown in Figs 4A-4D; and

Figs. 4A-4D illustrate the molecular structures of four different energy transfer dyes that can individually be used in four different dye sets, and include the structures for dyes 5-CFB-DR110-2 (Fig. 4A), 5-CFB-DR6G-2 (Fig. 4B), 6-CFB-DTMR-2 (Fig. 4C), and 6-CFB-DROX-2 (Fig. 4D).

It is to be understood that both the foregoing general description and the following detailed description are exemplary and explanatory only, and are intended to provide an explanation of various embodiments of the present teachings.

### DESCRIPTION

According to various embodiments, then detection system comprises two or more excitation sources, at least one detector, and a set of dyes. The two or more excitation sources can be adapted to emit a plurality of different individual excitation beam wavelength ranges, wherein each excitation source emits at least one wavelength that is not emitted in the excitation beam wavelength range of at least one other of the excitation sources. Each excitation source can comprise a respective individual radiation source or two or more excitation sources can together be provided by the same radiation source. For example, each excitation source can comprise a separate light-emitting diode (LED) or laser source, or two or more excitation sources can comprise a common broad spectrum light source, for example, a halogen lamp or other while light source, and appropriate optics, filters, gratings, or the like. A layered OLED or an LED array can provide two or more excitation sources. In an exemplary embodiment, a first excitation source can be provided that is adapted to emit a first excitation beam wavelength range of from about 460 nm to about 475 nm, and a second excitation source can be provided that is adapted to emit a second excitation beam wavelength range of from about 480 nm to about 495 nm. The second excitation beam wavelength range can be emitted at a different time or in a different direction than the first excitation beam wavelength range. In some embodiments, a group of excitation sources is provided that is adapted to emit two, three, four, or more, different and non-overlapping excitation beam wavelength ranges,

In some embodiments, the detection system can comprise a set of dyes and one or more of a sequence detection system, an electrophoretic detection system, a capillary electrophoresis system, a gel or slab electrophoresis system, a polymerase chain reaction (PCR) detection system, a real-time PCR detection system, a combination thereof, or the like.

As used herein, the term "spectrally resolvable" in reference to non-energy transfer dyes and donor dye moieties of energy transfer dyes can refer to different dyes having absorbances that are sufficiently distinct, that is, substantially non-overlapping, such that the absorption spectra of the respective dyes can be resolved using ordinary detection means, for example, a UV-vis spectrophotometer.

As used herein, the term "peak wavelength" in reference to excitation and emission can refer to a peak wavelength or a peak wavelength range that provides a maximum absorption of energy in the case of excitation, or a maximum energy release as radiation emitted from the dye, in the case of emission. The term "same wavelength" in reference to excitation and emission can refer to being at the exact same or at about the same peak wavelength or having substantially overlapping peak wavelength ranges. The term "different wavelength" in reference to excitation and emission can refer to wavelengths that are not exactly the same or that are not at about the same peak wavelength or that do not have substantially overlapping peak wavelength ranges. An example of a dye having a different excitation wavelength and emission wavelength is a spectrally resolvable dye. An example of different wavelengths can include excitations wavelengths of corresponding donor dye moieties that overlap by no more than about 50% with respect to each of the other donor dye moieties used in a set. The overlap of different wavelengths, if there is any overlap at all, can be by no more than about 10%, no more than about 5%, or no more than about 1%. In some embodiments, non-(energy transfer) dyes in a set can absorb light at different wavelengths than other dyes, for example, than energy transfer dyes, within the set. It will be understood, however, that it is not necessary for all non-(energy transfer) dyes and/or all energy transfer dyes of a set to absorb light in different wavelength ranges.

As used herein, the term "emission filter" can refer to any device adapted to provide a separation of emission beams from one or more dyes of a set. An emission filter can comprise a filter, a bandpass filter, a grating, a beam splitter, or the like.

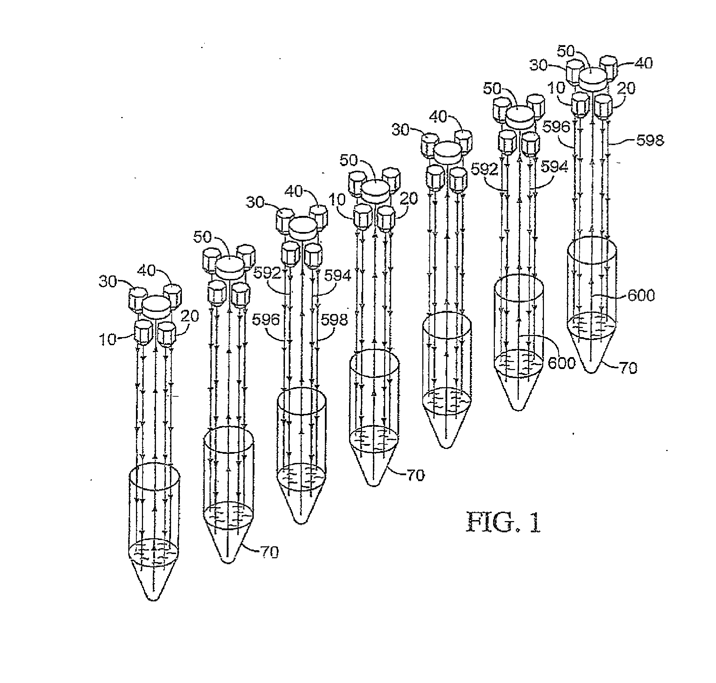

With reference to the drawings, Fig. 1 illustrates an exemplary system for nucleic acid sequence detection using PCR in a stationary volume that is thermally cycled. Excitation sources 10, 20, 30, and 40 can each provide radiation of a different peak wavelength and/or different peak wavelength range. For example, excitation source 10 can emit radiation depicted as beams 592 at a first wavelength of about 540 nm, excitation source 20 can emit radiation depicted as beams 594 at a second wavelength of about 560 nm, excitation source 30 can emit radiation depicted as beams 596 at a third wavelength of about 580 run, and excitation source 40 can emit radiation depicted as beams 598 at a fourth wavelength of about 600 run, The excitation beams emitted from excitation sources 10, 20, 30, and 40 can be directed to liquid sample portions 70 disposed in respective vials. Liquid sample portions 70 can each comprise nucleic acids to be detected and a set of dyes wherein the set of dyes comprises dyes that are respectively excitable by the respective excitation wavelengths emitted from excitation sources 10, 20, 30, and 40. For example, four energy transfer dyes can be selected wherein each one comprises a different respective donor dye moiety that is excitable by a respective one of the four different excitation sources 10, 20, 30, and 40. The acceptor dye moiety of each of the four energy transfer dyes can be selected to be the same for each energy transfer dye, for example, such that each dye emits radiation at the same wavelength upon excitation. Emission beams 600 emitted from the samples, for example, at an emission wavelength of about 620 nm, can be detected by detectors 50. In some embodiments, using a set of dyes wherein each dye of the set emits the same peak emission wavelength, for example, 620 nm, can be beneficial. In such a case the detector can be tuned for optimum detection at 620 nm, or at whatever emission wavelength is shared by two or more dyes of the set.

In some embodiments, the system shown in Fig. 1 can include one or more lenses (not shown) to focus excitation beams irradiated by the excitation sources, and/or one or more mirrors (not shown) to direct the excitation beams. In a system as shown in Fig. 1 that comprises multiple radiation sources as the multiple excitation sources, the excitation wavelengths and emission wavelength can be selected so that they are all different and spectrally spaced or separated from one another, resulting in a system that does not require filters to reduce or eliminate overlapping radiation.

In some embodiments, excitation sources 10, 20, 30, and 40 illustrated in Fig. 1 can comprise four individual LEDs. In some embodiments, the excitation sources can be integrated together, for example, in the form of a two-layer OLED comprising two layers of respectively different OLEDs that can provide two different and spectrally spaced respective excitation wavelengths. In some embodiments, any of the various radiation sources described herein can be used as excitation sources 10, 20, 30, and 40.

In some embodiments, the system illustrated in Fig. 1 can provide independent control for each of excitation sources 10, 20, 30, and 40, for example, control by a CPU, other processor, or by logic. The processor can comprise, for example, a computer programmed to actuate the excitation sources one at a time. The processor can comprise instrumentation control circuitry therein. The processor can be coupled to the detector to coordinate and/or correlate an actuated excitation source with an emission wavelength, an emission wavelength range, or an emission spectra, detected by the detector. The excitation source actuated can be correlated to the dyes used in the system to detect nucleic acid sequences.

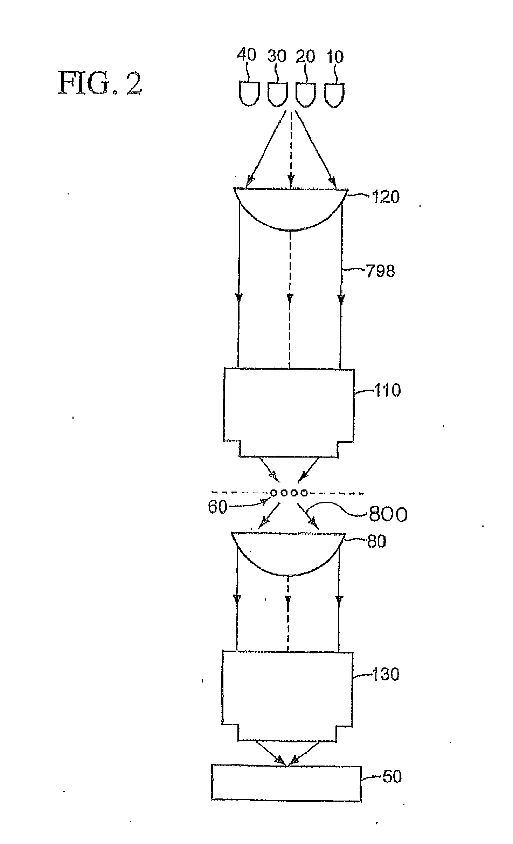

Fig. 2 illustrates an exemplary system according to various embodiments for nucleic acid sequencing using capillary electrophoresis. Excitation sources 10, 20, 30, and 40 shown in Fig. 2 can each provide excitation beams 798 of different peak excitation wavelengths and/or different peak excitation wavelength ranges. Excitation beams 798 can be collimated by collection lens 120 and focused on capillaries 60 by lens 110. The capillaries 60 include the nucleic acids to be detected and the energy transfer dyes selected to be excitable by the wavelengths provided by excitation sources 10, 20, 30, and 40. For example, four energy transfer dyes can be selected with each having a respective donor dye moiety that is excitable by a different one of excitation sources 10, 20, 30, and 40, respectively. The acceptor dye moieties can be selected to be the same for each of the four transfer dyes, for example, such that each of the dyes emits emission beams at the same wavelength. As illustrated, emission beams 800 can be collimated by collection lens 80 and imaged onto detector 50 by imaging lens 130. In some embodiments, the system can comprise, for example, separate LEDs or layered OLEDs as excitation sources 10, 20, 30, and 40.

In some embodiments, the system illustrated in Fig, 2 can provide independent control for each of excitation sources 10, 20, 30, and 40, for example, control by a CPU or other processor. The processor can comprise, for example, a computer programmed to actuate the excitation sources one at a time. The processor can comprise instrumentation control circuitry therein. The processor can be coupled to the detector to coordinate and/or correlate an actuated excitation source with an emission wavelength, an emission wavelength range, or an emission spectra, detected by the detector. The processor can control an alternate or sequential operation or cycling of excitation sources 10, 20, 30, and 40. The excitation source actuated can be correlated to the particular dye of the set and used to determine the sequence of a nucleic acid.

In some embodiments, the present teachings provide for a set of dyes wherein at least two dyes of the set have different respective absorption wavelengths and the same or substantially overlapping emission wavelengths. As used herein, the term "set of dyes" can refer to any combination of 2, 3, 4, or more dyes comprising non-(energy transfer) and/or energy transfer dyes. In some embodiments, the present teachings provide a set of dyes wherein at least one of the dyes of the set is an energy transfer dye. In some embodiments, the wavelength range for emission of at least one of the dyes of the set will be the same as or will substantially overlap with the wavelength range for emission of at least one other dye of the set. In some embodiments, the wavelength range for absorption of each of the dyes of the set will be spectrally resolvable relative to the wavelength ranges for absorption of the other dyes of the set.

Suitable dyes for use in connection with the present teachings can be any of a variety of dyes that are known in the art including, but not limited to, rhodamines, fluoresceins, rhodols, pyronines, carbopyronines, coumarins, porphyrins, including but not limited to phthalocyanines, metaloporphyrins, and lanthanide complexes, cyanines, hemicyanines, squaric acids, merocyanines, bodipys, phenoxazines, including but not limited to oxonines, resorufamines and resorufins, acridines, aminoacridines, carbazines, phenotliaziums, pyryliums, and the like.

Examples of suitable dyes for use in connection with the present teachings include any of those described in, for example, Menchen, et al., U,S. Patent No. 5,188,934; Benson, et al., U,S. Patent No. 6,020,481; Lee, et al,, U.S. Patent No. 5,847,162; Benson, et al., U.S. Patent No. 6,008,379; Benson, et al., U.S. Patent No. 5,936,087; Upadhya, et al., U.S. Patent No. 6,221,604; Lee, et al., U.S. Patent No. 6,191,278; Yan, et al., U.S. Patent No. 6,140,500; Mao, et al., U.S. Patent No. 6,130,101; Glazer, et al., U.S. Patent No. 5,853,992; Brush, et al., U.S. Patent No. 5,986,086; Hamilton, et al., U.S. Patent No. 6,140,494; and Hermann, et al., U.S. Patent No. 5,750,409, each of which is incorporated by reference in its entirety with regard to fluorescent dye structures, fluorescent dye synthesis, fluorescent dye conjugation to biopolymers, application of fluorescent dyes in energy transfer dyes, and fluorescent dye spectral properties. Example of suitable dyes for use in connection with the present teachings include, but are not limited to, 5-carboxyfluorescein, 6-carboxyfluorescein, rhodamine green (R110), 5-carboxyrhodamine, 6-carboxyrhodamine, N,N'-diethyl-2',7'-dimethyl-5-carboxy-rhodamine (5-R6G), N,N'-diethyl-2',7'-dimethyl-6-carboxyrhodamine (6-R6G), N,N,N',N'-tetramethyl-5-carboxyrhodamine (5-TAMRA), N,N,N,N'-tetramethyl-5-carboxyrhodamine (6-TAMRA.), 5-carboxy-X-rhodamine (5-ROX), 6-carboxy-X-rhodamine (6-ROX), 5-carboxy-2',4',5',7',-4,7-hexachlorofluorescein, 6-carboxy-2',4',5',7',4,7-hexachloro-fluoreseein, 5-carboxy-2',7'-dicarboxy-4',5'-dichlorofluorescein, 6-carboxy-2',7'-dicarboxy-4',5'-dichlorofluoreseein, 5-carboxy-2',4',5',7'-tetrachlorofluorescein, 1',2'-benzo-4'-fluoro-7',4,7-triehloro-5-carboxyfluorescein, 1',2'-benzo-4'-fluoro-7',4,7-trichloro-6-carboxy-fluorescein, 1 ',2',7',8'-dibenzo-4,7-dichloro-5-carboxyfluorescein, as well as other commercially available dyes as shown in Table 1 below.

**TABLE 1**

| **Fluorescent Dye** | **Absorbance** | **Emission** | **Extinction** |
|---|---|---|---|
| | **(nm)** | **(nm)** | **Coefficient** |
| 5-Fluorescein | 495 | 520 | 73000 |
| 5 -Carboxyfluorescein (5-FAM) | 495 | 520 | 83000 |
| 6 -Carboxyfluorescein (6-FAM) | 495 | 520 | 83000 |
| 6-Carboxyhexachlorofluoresecin (6-HEX) | 535 | 556 | 73000 |
| 6-Carboxytetrachlorofluofescein (6-TET) | 521 | 536 | 73000 |
| JOE | 520 | 548 | 73000 |
| LightCycler Red640 | 625 | 640 | |
| LightCycler Red 705 | 685 | 705 | |
| Oregon Green 488 | 496 | 516 | 76000 |
| Oregon Green 500 | 499 | 519 | 84000 |
| Oregon Green 514 | 506 | 526 | 85000 |
| BODIPY FL-X | 504 | 510 | 70000 |
| BODIPY FL | 504 | 510 | 70000 |
| BODIPY-TMR-X | 544 | 570 | 56000 |
| BODIPY R6G | 528 | 547 | 70000 |
| BODIPY 650/665 | 650 | 665 | 101000 |
| BODIPY 564/570 | 563 | 569 | 142000 |
| BODIPY 581/591 | 581 | 591 | 136000 |
| BODIPY TR-X | 588 | 616 | 68000 |
| BODIPY 630/650 | 625 | 640 | 101000 |
| BODIPY 493/503 | 500 | 509 | 79000 |
| 5-Carboxyrhodamine 6G | 524 | 557 | 102000 |
| 5(6)-Carboxytetramethylrhodamine (TAMRA) | 546 | 576 | 90000 |
| 6-Carboxytetramethylrhodamine (TAMRA) | 544 | 576 | 90000 |
| 5(6)-Carboxy-X-Rhodamine (ROX) | 576 | 601 | 82000 |
| 6-Carboxy-X-Rhodamine (ROX) | 575 | 602 | 82000 |
| AMCA-X (Coumarin) | 353 | 442 | 19000 |
| Texas Red-X | 583 | 603 | 116000 |
| Rhodamine Red-X | 560 | 580 | 129000 |
| Marina Blue | 362 | 459 | 19000 |
| Pacific Blue | 416 | 451 | 37000 |
| Rhodamine Green-X | 503 | 528 | 74000 |
| 7-diethylaminocoumarin-3-carboxylic acid | 432 | 472 | 56000 |
| 7-methoxycoumarin-3-carboxylic acid | 358 | 410 | 26000 |
| Cy3 | 552 | 570 | 150000 |
| Cy3B | 558 | 573 | 130000 |
| Cy5 | 643 | 667 | 250000 |
| Cy5.5 | 675 | 694 | 250000 |
| DY-505 | 505 | 530 | 85000 |
| DY-550 | 553 | 578 | 122000 |
| DY-555 | 555 | 580 | 100000 |
| DY-610 | 606 | 636 | 140000 |
| DY-630 | 630 | 655 | 120000 |
| DY-633 | 630 | 659 | 120000 |
| DY-636 | 645 | 671 | 120000 |
| DY-650 | 653 | 674 | 77000 |
| DY-675 | 674 | 699 | 110000 |
| DY-676 | 674 | 699 | 84000 |
| DY-681 | 691 | 708 | 125000 |
| DY-700 | 702 | 723 | 96000 |
| DY-701 | 706 | 731 | 115000 |
| DY-730 | 734 | 750 | 113000 |
| DY-750 | 747 | 776 | 45700 |
| DY-751 | 751 | 779 | 220000 |
| DY-782 | 782 | 800 | 102000 |
| Cy3.5 | 581 | 596 | 150000 |
| EDANS | 336 | 490 | 5700 |
| WellRED D2-PA | 750 | 770 | 170000 |
| WellRED D3-PA | 685 | 706 | 224000 |
| WellRED D4-PA | 650 | 670 | 203000 |
| Pyrene | 341 | 377 | 43000 |
| Cascade Blue | 399 | 423 | 30000 |
| Cascade Yellow | 409 | 558 | 24000 |
| PyMPO | 415 | 570 | 26000 |
| Lucifer Yellow | 428 | 532 | 11000 |
| NBD-X | 466 | 535 | 22000 |
| Carboxynapthofluorescein | 598 | 668 | 42000 |
| Alexa Fluor 3 5 0 | 346 | 442 | 19000 |
| Alexa Fluor 405 | 401 | 421 | 35000 |
| Alexa Fluor 430 | 434 | 541 | 16000 |
| Alexa Fluor 488 | 495 | 519 | 71000 |
| Alexa Fluor 532 | 532 | 554 | 81000 |
| Alexa Fluor 546 | 566 | 573 | 104000 |
| Alexa Fluor 555 | 555 | 565 | 150000 |
| Alexa Fluor 568 | 578 | 603 | 91300 |
| Alexa Fluor 594 | 590 | 617 | 73000 |
| Alexa Fluor 633 | 632 | 647 | 100000 |
| Alexa Fluor 647 | 650 | 665 | 239000 |
| Alexa Fluor 660 | 663 | 690 | 132000 |
| Alexa Fluor 680 | 679 | 702 | 184000 |
| Alexa Fluor 700 | 702 | 723 | 192000 |
| Alexa Fluor 750 | 749 | 775 | 240000 |
| Oyster 556 | 556 | 570 | 155000 |
| Oyster 645 | 645 | 666 | 250000 |
| Oyster 656 | 656 | 674 | 220000 |
| 5(6)-Carboxyeosin | 521 | 544 | 95000 |
| Erythrosin | 529 | 544 | 90000 |

Suitable dyes for use in connection with the present teachings also include energy transfer dyes. As used herein, "energy transfer dye" includes any dye that comprises a donor dye covalently attached to an acceptor dye through a linker moiety, such that the donor dye is capable of absorbing light at a first wavelength or wavelength range and emitting excitation energy in response, and the acceptor dye is capable of absorbing the excitation energy emitted by the donor dye and fluorescing at a second wavelength or wavelength range in response.

Suitable energy transfer dyes for use in connection with the present teachings can include energy transfer dyes that comprise any pair of the dyes listed above having spectral properties that are consistent with the above definition. Examples of energy transfer dyes suitable for use in connection with the present teachings include, but are not limited to those described in, for example, Mathies, et al. U.S. Patent No. 5,728,528, Lee, et al. U.S. Patent No. 5,863,727, Glazer, et al. U.S. Patent No. 5,853,992, Waggoner, et al., U.S. Patent No. 6,008,373, Nampalli, et al., U.S. Patent Application Pub. No. 2004/0126763 A1, Kumar, et al., PCT Pub. No. WO . 00/13026A1, and PCT Pub. No. WO 01/19841A1, each of which is incorporated herein by reference for all it discloses with regard to energy transfer dye structures, energy transfer dye synthesis, energy transfer dye linkers, alternative donor dyes, alternative acceptor dyes and energy transfer dye spectral properties.

Energy transfer dyes generally can provide a larger effective Stokes shift than non-energy transfer fluorescent dyes because the Stokes shift of an energy transfer dye is determined by the difference between the wavelength at which the donor dye maximally absorbs light and the wavelength at which the acceptor dye maximally emits excitation energy. It will be appreciated by those of skill in the art that by knowing the Stokes shift of various possible donor dyes and acceptor dyes, it can be possible to prepare energy transfer dyes having a desired maximal absorption wavelength, a desired maximal emission wavelength and a desired Stokes shift.

Although fluorescent dyes are described as labels for the detection of analytes, it is to be understood that QdotⓇ bioconjugates can be used instead of, or in addition to, the described dyes. Accordingly, it is to be understood that where the use of a fluorescent dye is described herein, a QdotⓇ bioconjugate can be substituted therefore. A QdotⓇ bioconjugate is a term that can be used to describe QdotⓇ nanocrystals coupled to proteins, oligonucleotides, small molecules, etc., which can be used to direct binding of quantum dots to targets of interest. Examples of QdotⓇ bioconjugates can include streptavidin, protein A, and biotin families of conjugates. Further information on QdotⓇ bioconjugates can be found in M. P. Bruchez et, al., Semiconductor nanocrystals as fluorescent biological labels, Science 1998, 281, 2013, and in U.S. Patent Application Publication No. 2005/0141843, that is incorporated herein, in its entirety, by reference. QdotⓇ bioconjugates can be obtained from the Invitrogen Corporation, 1600 Faraday Avenue, Carlsbad, California 92008.

It will be understood by one of skill in the art that the covalent attachment (i.e.-conjugation) of fluorescent dyes and fluorescent energy transfer dyes to biomolecules, including polynucleotides, is well known in the art. For example, the conjugation of a dye or an energy transfer dye to a biomolecule or the conjugation of one dye or dye moiety to another dye or dye moiety can be achieved by formation of the N-hydroxysuccinimidyl ester (NHS ester) of the dye or energy transfer dye followed by subsequent reaction of the NHS ester with an amine group on a linker that is covalently attached to a biomolecule or a linker that is covalently attached to another dye. Numerous examples of and methods for the conjugation of dyes and energy transfer dyes, or moieties thereof, to linkers and biomolecules are given in the references cited above.

In some embodiments, a plurality of dyes including at least one energy transfer dye can be included in a dye set. Each of the one or more energy transfer dye of the set can include a donor dye moiety, an acceptor dye moiety, and a linker. The donor dye moiety can be capable of absorbing radiation at a first wavelength or within a first wavelength range, and emitting excitation energy, at a second, different wavelength or within a second wavelength range, while the acceptor dye moiety can be capable of absorbing the excitation energy that is emitted from the donor dye moiety and fluorescing in response to the excitation.

In some embodiments, a set of dyes comprising two or more energy transfer dyes can be provided. Each energy transfer dye of the set can include a spectrally resolvable donor dye moiety, relative to the other dyes of the set, and an acceptor dye moiety that is the same as or has an emission wavelength or emission wavelength range that is the same as, or that substantially overlaps with, the emission wavelengths or emission wavelength ranges of the other acceptor dye moieties of the set. It will be understood that by including the same acceptor dye moiety or an acceptor dye moiety having the same or a substantially overlapping emission wavelength, in each energy transfer dye of the set, the energy transfer dyes can have emission at a common wavelength range but absorption in spectrally resolvable wavelength ranges. As used herein, the terms "substantially overlap", "substantially overlaps" and "substantially overlapping", when used to describe overlapping emission and/or absorption wavelengths of fluorescent dyes, means that the wavelength ranges overlap by at least about 50%.

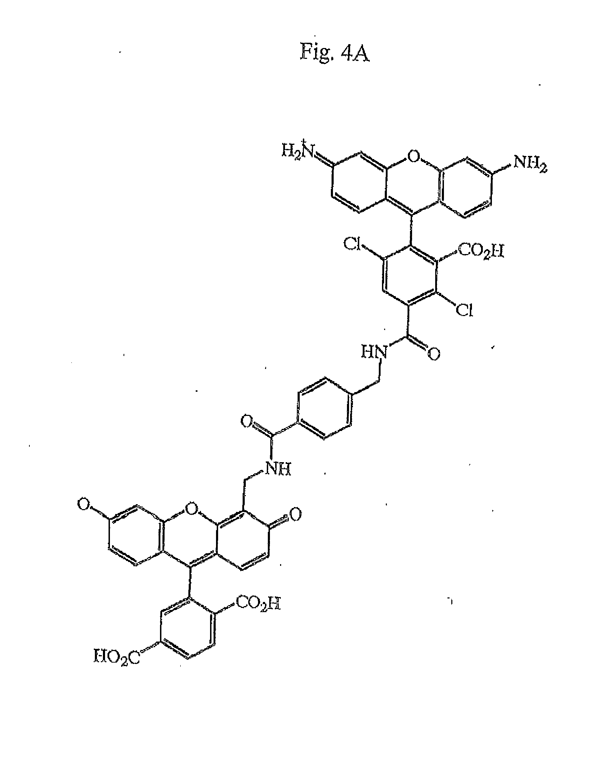

In some embodiments, a set of dyes can include one or more energy transfer dye and one or more non-energy transfer dye. The one or more non-energy transfer dye can comprise, for example, one or more dye selected from 5-carboxyfluorescein (5-FAM), 6-carboxyfluorescein (6-FAM), rhodamine green (RII0), 5-carboxyrhodamine, 6-carboxyrhodamine, N,N'-diethyl-2',7'-dimethyl-5-carboxyrhodamine (5-R6G), N,N'-diethyl-2',7'-dimcthyl-6-carboxyrhodamine (6-R6G), N,NN',N'-tetramethyl-5-carboxyrhodamine (5-TAMRA), N,N,N',N'-tetramethyl-5-carboxyrhodamine (6-TAMRA), 5-carboxy-X-rhodamine (5-ROX), 6-carboxy-X-rhodamine (6-ROX), 5-carboxy-2',4',5',7',-4,7-hexachloro-fluorescein, 6-carboxy-2',4',5',7',4,7-hexachlorofluorescein, 5-carboxy-2',7'-dicarboxy-4',5'-dichlorofluorescein, 6-carboxy-2',7'-dicarboxy-4',5'-dichlorofluorescein, 5-carboxy-2',4' ,5' ,7' -tetrachlorofluorescein, 1',2'-benzo-4'-fluoro-7',4,7-trichloro-5-carboxy-fluorescein, 1',2'-benzo-4'-fluoro-7',4,7-trichloro-6-carboxyfluorescein, 1,2',7',8'-dibenzo-4,7-dichloro-5-carboxyfluorescein, as well as other commercially available dyes as shown in Table 1 above. Suitable energy transfer dyes can include, but are not limited to, those shown in Figures 4A-4D as well as energy transfer dyes that comprise some combination including one or more donor dyes moieties or moieties selected from 5-carboxyfluorescein (5-FAM), 6-carboxyfluorescein (6-FAM), rhodamine green (R110), 5-carboxyrhodamine, 6-carboxyrhodamine and Cy3 with the acceptor dyes N,N'-diethyl-2',7'-dimethyl-5-carboxyrhodamine (5-R6G), N,N'-diethyl-2',7'-dimethyl-6-catboxyrhodamine (6-R6G), N,N,N',N'-tetramethyl-5-carboxyrhodamine (5-TAMRA), N,N,N',N'-tetramethyl-5-carboxyrhodamine (6-TAMRA), 5-caxboxy-X-rhodamine (5-ROX), 6-carboxy-X-rhodamine (6-ROX), Cy3.5, Cy5, Cy5.5 and Cy7. It will be understood that further energy transfer dye combinations are possible, based on the spectral properties desired, by choosing donor dye moieties and an acceptor dye moiety from, for example, Table 1, such that the emission wavelength ranges of the donor dye moieties of the dyes of the set are the same as or substantially overlap with the emission wavelength ranges of the other respective acceptor dye moieties of the dyes of the set.

In some embodiments, a set of dyes can include two or more different subsets of dyes according to the present teachings, Such sets of dyes can be used together to detect an even greater number of different analytes in a sample. For example, two sets of two dyes can be provided wherein each set of two dyes comprises two non-energy transfer dyes, two energy transfer dyes, or one energy transfer dye plus one non-energy transfer dye. In each set, both dyes of the set, whether they comprise non-energy transfer dyes, energy transfer dyes, or both, can have the same or a substantially overlapping emission wavelength range, and both dyes of each set can have spectrally resolvable absorption wavelength ranges,

Using the dyes illustrated in Table 1 as an example, a set of dyes can be created comprising three energy transfer dyes and one non-energy transfer dye. In such a set each of the three energy transfer dyes can have a ROX moiety as the acceptor dye moiety and each energy transfer dye can have one of a FAM moiety, a TET moiety, or a TAMRA moiety, respectively, as the donor dye moiety. This arrangement can provide a set of three energy transfer dyes having spectrally resolvable absorption wavelengths and the same emission wavelength. In addition to the three energy transfer dyes, the set can also include ROX as the non-energy transfer dye, in which case, all four dyes of the set will have spectrally resolvable absorption wavelengths and the same emission wavelength.

In another example, a set of dyes comprising subsets of dyes having the same emission wavelength within the subset can be created. For example, in one subset, a Cy5 moiety could serve as the as the acceptor dye moiety in an energy transfer dye where a FAM moiety is the donor dye moiety, and Cy5 could serve as a non-energy transfer dye to provide a set of two dyes each having a Cy5 emission wavelength but each having a spectrally resolvable absorption wavelength relative to the other dye of the set. The set could also include a subset where a ROX moiety serves as the acceptor dye moiety for energy transfer dyes comprising either a TET moiety or a TAMRA moiety, respectively, as the donor dye moiety, and ROX could serve as the non-energy transfer dye of the set. As a result, a set of 5 dyes could be envisioned having two sets of spectrally resolvable emission wavelengths (e.g., ROX and Cy5) and a set of 5 spectrally resolvable absorption wavelengths.

It will be understood that many other dyes and energy transfer dyes could be used in addition to or in place of each of the dyes in the preceding examples. Furthermore, energy transfer dyes of the type described in the preceding examples can be made in accordance with the teachings of Lee, et al., U.S. Patents Nos. 5,800,996, 5,863,727, and 5,945,526, which are incorporated herein in their entireties by reference.

In some embodiments, a plurality of energy transfer dyes can be provided in a set of energy transfer dyes, wherein each of the donor dye moieties of the plurality of energy transfer dyes absorbs radiation at a different wavelength or in a different wavelength range when compared to the other dyes of the set. The acceptor dye moieties of the respective energy transfer dyes of the set can all have the same structure or have a substantially overlapping peak emissions wavelength range as the other acceptor dye moieties of the set.

In some embodiments, each acceptor dye moiety of the plurality of energy transfer dyes of the set is different from at least one other acceptor dye moiety but each acceptor dye moiety of the plurality of energy transfer dyes emits radiation within the same peak emission wavelength range or within an emission wavelength range that overlaps by, or that is overlapped by, at least about 50% of the emission wavelength range of at least one of the other acceptor dye moieties used in the set,

In some embodiments, the acceptor dye moieties of the respective energy transfer dyes of the set can emit radiation at the same emission wavelength range, for example, each acceptor dye moiety can emit radiation within a first emission wavelength range and all of the peak emission wavelength ranges overlap a detectable wavelength range of a detector, for example, such that each emits a peak wavelength that falls within the same 20 nm-wide band or 10 nm-wide band of wavelengths. Although a 20 nm-wide band and a 10 nm-wide band are exemplified, other widths of wavelength bands can be selected. The respective emissions can occur in response to respective excitation of the respective donor dye moieties of the energy transfer dyes

A kit, or a set of dyes, according to the present teachings can be useful in numerous applications, and provided, for example: as labeled nuclcotides or as labeled nucleic acid sequences; as labels covalently attached to terminator nucleotides in DNA sequencing reactions; as labels covalently attached to PCR primers; as labels covalently attached to PCR probes; combinations thereof; and the like. In some embodiments, the dyes can be provided as such labels. In some embodiments, the kits can comprise a mixture of such labels, together in a single tube, vial, or other container. If used as labels attached to nucleotides, the labels can react with and bond, for example, with complementary nucleic acid sequences in a sample, for example, with DNA molecules. For example, in some embodiments the present teachings provide for a set of dyes, optionally comprising at least one energy transfer dye, wherein each dye of the set is covalently attached to a polynucleotide probe and the polynucleotide probe is covalently attached to at least one quencher moiety. The fluorescence of each dye can be substantially quenched by the quencher moiety while the probe is intact. In some embodiments, the probe can be cleaved, for example, by a polymerase having 5'-exonuclease activity, thus causing separation of the quencher moiety from the remaining dye structure of the dye and resulting in enhanced fluorescence emission from the remaining dye structure of the previously quenched dye. In some embodiments, the quencher moiety can comprise a moiety of a non-fluorescent quencher dye that can have low background fluorescence. Exemplary quencher dyes, energy transfer dyes, donor dyes, acceptor dyes, and moieties thereof, which can be used in various dye sets according to various embodiments, are described, for example, in U.S. Patent No. 5,876,930 to Livak et al., and in U.S. Patent No. 5,863,727 to Lee et al., which are incorporated herein in their entireties by reference. Other quencher moieties can be used, for example, hybridization quenchers such as those described in U.S. Patent No. 6,750,024 which is incorporated herein in its entirety by reference.

In some embodiments, at least one radiation source is provided that can provide at least two excitation sources and emit radiation at, at least two different respective wavelengths, for example, at, at least two different, spaced-apart and non-overlapping wavelength ranges. One, two, or more excitation sources can be used. The at least two different wavelengths or wavelength ranges can be irradiated at a sample separately to first detect a first analyte and then to second detect a second analyte. In some embodiments, the two excitation sources can comprise a single light source, for example, a white light source including wavelengths of different visible light colors. If a single source is used, one or more filters, prisms, diffraction gratings, other spectra-separating devices, or a combination thereof, can be used to independently provide radiation of a first peak wavelength range and radiation of a different, second peak wavelength range.

According to various embodiments, detection can be accomplished, for example, when two analytes to be detected are attached to two respective dyes and one of the dyes exhibits a relative increase in fluorescence when irradiated with radiation at the first peak wavelength range while the other dye exhibits a relative increase in fluorescence when irradiated with radiation at the second peak wavelength range. In such a system, a single detector can be used to detect emissions from each dye and the two analytes can be distinguished from one another based on the excitation source used to cause the detectable emission. A plurality of different analytes in a single sample can be detected, depending on the number of different dyes of the set, and detection is not limited to determining the presence of only one or two analytes in a sample.

In some embodiments, detection can be accomplished using photodetection systems, components, and/or techniques, for example, using excitation and/or detection systems and/or components of sequence detection or sequencing based on PCR as known in the art of nucleic acid sequence analysis.

In some embodiments, a system is provided that includes a plurality of excitation sources each being capable of providing beams of radiation at a specific wavelength, and a set of dyes wherein at least one dye of the set can absorb or be excited at the same wavelength provided by a first one of the plurality of excitation sources. In some embodiments, a second excitation source of the plurality of excitation sources provides a second specific wavelength and/or peak wavelength range such that a second dye can be excited by the second excitation source, but not from the first excitation source, and not from the radiation emitted by the first dye as a result of excitation of the first dye. Furthermore, at least one of the additional excitation sources can provide a third excitation wavelength, for example, while providing little or no radiation in specific wavelengths of either the first excitation source or of the second excitation source.

In some embodiments, the presence of energy transfer dyes in a sample can be used to identify various components or analytes present in the sample. The energy transfer dyes of the set can be designed or selected such that each energy transfer dye results in (a) a discrete absorption at a first wavelength, and (b) a discrete emission at a second wavelength.

In some embodiments, each energy transfer dye used can comprise a donor dye moiety capable of both absorbing light at a first wavelength and emitting excitation energy at a second wavelength, and an acceptor dye moiety capable of both absorbing excitation energy emitted by the donor dye moiety at the second wavelength and emitting radiation at a third wavelength. Each energy transfer dye can optionally also comprise a linker moiety that links the donor dye moiety and the acceptor dye moiety together. In some embodiments, each donor dye moiety of a plurality of energy transfer dyes absorbs maximum radiation at a different wavelength than the other energy transfer dyes used in the system, for example, mixed with a sample. Each acceptor dye moiety of the respective energy transfer dyes can emit maximum radiation at the same wavelength as the other energy transfer dyes in the sample.

In some embodiments, each respective donor dye moiety of the plurality of energy transfer dyes can include a different dye moiety, and each respective acceptor dye moiety of the plurality of energy transfer dyes can include the same dye moiety or a respective dye moiety that emits maximum radiation at the same or at about the same wavelength as the other acceptor dye moieties of the set.

In some embodiments, a system is provided that includes two or more excitation sources, one or more detector, and an appropriate set of dyes. The set of dyes can comprise one or more energy transfer dyes.

In some embodiments, the system can include one or more excitation or radiation sources, for example, one or more lasers, one or more solid state lasers, one or more laser diodes, one or more nanotube field emitter light sources, one or more light-emitting diodes ("LED"), one or more organic light-emitting diodes ("OLED"), one or more thin film electroluminescent devices ("TFELD"), one or more quantum dot LEDs, and/or one or more phosphorescent OLEDs ("PHOLED"). Examples of suitable LEDs, OLEDs, quantum dot LEDs, and solid state lasers in various configurations are known in the art of fluorometry, In some embodiments, the excitation source can include a plurality of excitation sources that can emit maximum radiation of different respective peak wavelengths and/or peak wavelength ranges.

In some embodiments, the system can include one or more detectors, for example, a single wavelength detector, a multiple wavelength detector, a multiplex detector, a photodiode array, a change-coupled diode array, a camera, a CMOS detector, and/or any combination thereof. The one or more detectors can be operably arranged to receive radiation emitted from the different dyes of the set, upon respective excitation of the different dyes.

In some embodiments, a kit is provided that can comprise a plurality of dyes, including at least one energy transfer dye as described herein, packaged together. Each energy transfer dye of the kit can include a donor dye moiety as described herein, an acceptor dye moiety as described herein, and optionally a linker moiety as described herein. In some embodiments, each of the donor dye moieties of the energy transfer dyes of the kit can absorb maximum radiation at a different peak wavelength and/or at a different peak wavelength range, when compared to the absorbance of at least one other dye of the kit. In some embodiments, each of the acceptor dye moieties of the energy transfer dyes of the kit can emit maximum radiation at the same or at about the same peak wavelength or peak wavelength range, when compared to the emission of at least one other dye of the kit. In some embodiments, the emission wavelength range of each dye of the set substantially overlaps with the emission wavelength range of at least one other dye in the kit.

The kit can be packaged in a box or other container and/or can be heat sealed, heat-wrapped, shrunk-wrapped, or otherwise enveloped or encapsulated. The kit can include a system for retaining the plurality of dyes, or a plurality of dye containers, for example, vials. For example, the kit can include a vial rack. The kit can include two or more dyes, including at least one energy transfer dye, each contained in the same or in a different respective separate container. The kit can include three or more dyes each contained in the same or in a different respective separate container. The kit can contain four or more dyes each contained in the same or in a different respective separate container. In some embodiments, at least one of the dyes of the kit can be an energy transfer dye as described herein. In some embodiments, at least two of the dyes of the kit can be energy transfer dyes as described herein.

In some embodiments, a plurality of the dyes of the kit, including at least one energy transfer dye, or all of the dyes of the kit, can be contained together in a single container, for example, in a single tube or vial. The single tube or vial can be labeled, for example, with a tag. The tag can comprise a barcode, a two-dimensional barcode, or a radio frequency identification tag. In some embodiments, a tag can be included in an outer package, for example, disposed in or on a box.

In some embodiments, the kit can also include one or more excitation sources, for example, one or more of the radiation sources described above, or an array of excitation sources. In some embodiments, the kit can include one or more detectors, for example, one or more monochromatic or single wavelength detectors, one or more multiple wavelength detectors, one or more multiplex detectors, one or more photodiodes, one or more photodiode arrays, one or more charge-coupled diode arrays, one or more cameras, one or more CMOS detectors, or one or more combinations thereof. The one or more detectors can include one or more avalanche photodiodes. The kit can include optics components, for example, mirrors, focusing lenses, Fresnel lenses, and the like. The kit can be used to modify existing systems that are adapted to be used with a set of dyes other than the sets disclosed herein.

In some embodiments, a method of detecting an analyte in a sample is provided and includes: providing a sample that contains at least two detectable dyes; irradiating the sample with first excitation beams; detecting first emission beams emitted by the dyes in the sample in response to irradiating the sample with the first excitation beams; irradiating the sample with second excitation beams that differ from the first excitation beams; and detecting second emission beams emitted from the dyes in the sample in response to irradiating the sample with the second excitation beams. The at least two detectable dyes can comprise a set of at least two respective fluorescent dyes, for example, including at least one energy transfer dye as described herein. Each detectable dye can be adapted to emit respective detectable emission beams when irradiated by an excitation beam that includes radiation of a respective peak wavelength useful for exciting the respective dye. For example, each dye can emit detectable radiation when irradiated with a respective peak wavelength that falls within the maximum absorption wavelength range of the respective dye. In some embodiments, the first emission beams detected and the second emission beams detected can be of the same wavelength or of substantially the same or substantially overlapping wavelength ranges.

In some embodiments, the method can include irradiating a sample with one or more excitation sources as described herein, for example, with one or more lasers, one or more solid state lasers, one or more laser diodes, one or more nanotube field emitter light sources, or one or more LEDs, one or more OLEDs, one or more TFELDs, one or more quantum dot-based LEDs, or one or more PHOLEDs.

In some embodiments, the method can include detecting emitted radiation with one or more detectors, for example, one or more single wavelength detectors, one or more multiple wavelength detectors, one or more multiplex detectors, one or more photodiode arrays, one or more charge-coupled diode arrays, one or more cameras, one or more CMOS detectors, or one or more combinations thereof.

### EXAMPLES

The present teachings will now be further explained in the following prophetic examples. The following examples are in no way meant to limit the present teachings and one of skill in the art will understand that many more embodiments beyond those exemplified below are possible.

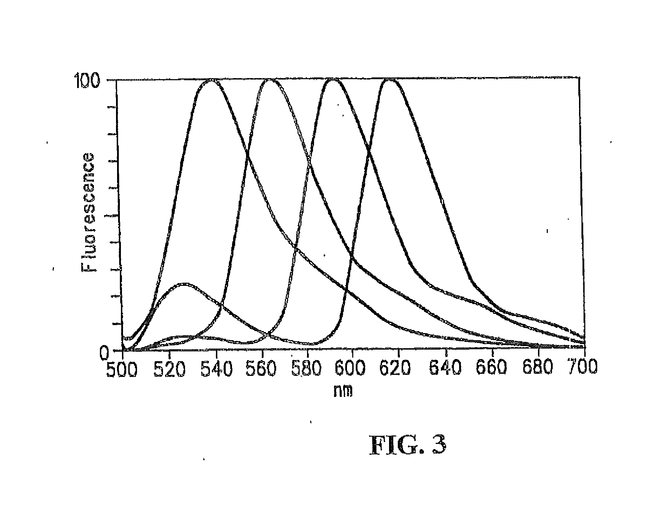

EXAMPLE 1 - Exemplary sets of dyes can include one or more energy transfer dyes in some embodiments, and can, for example, comprise one of the four energy transfer dyes of the structures shown in Figs. 4A-4D. Each exemplary energy transfer dye was attached to a 21-base length primer sequence with an aminohexyl linkage at the 5' end and the resulting complex was irradiated with excitation beams. The oligonucleotides were quantitated based on their respective absorbances at 260 nm, assuming an extinction coefficient of 180,000 cm⁻¹ M⁻¹. Spectra were obtained at a primer concentration of 0.4 µM in 8M urea, 1 X Tris/Borate/EDTA (TBE) buffer using a 488 nm excitation source. The respective structures of four energy transfer dyes illustrated in Figs. 4A-4D correspond to the structures that generated the four peaks shown from left to right in Fig. 3, respectively.

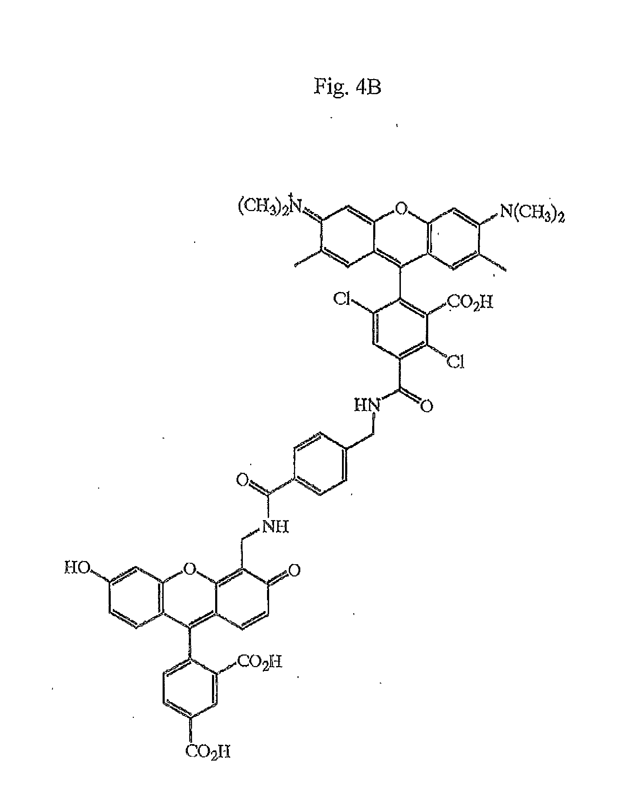

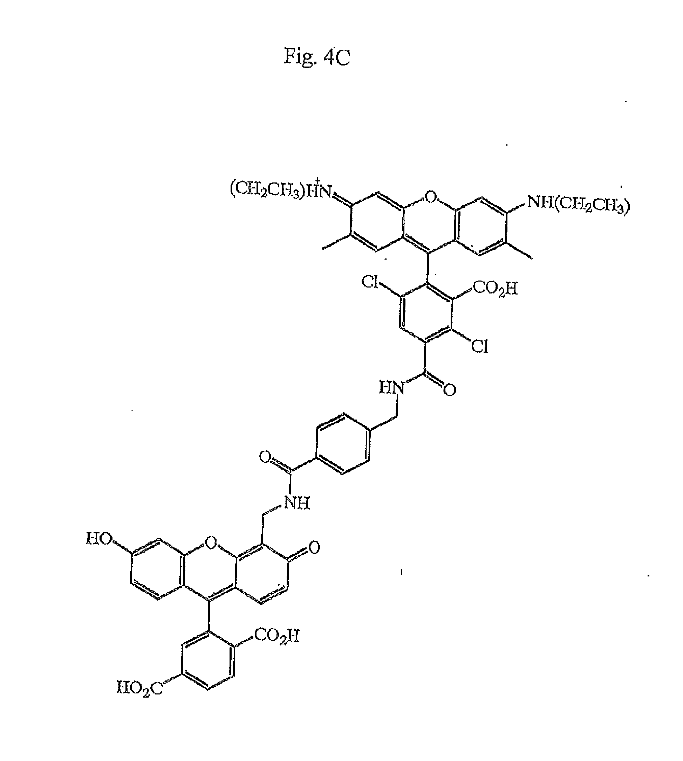

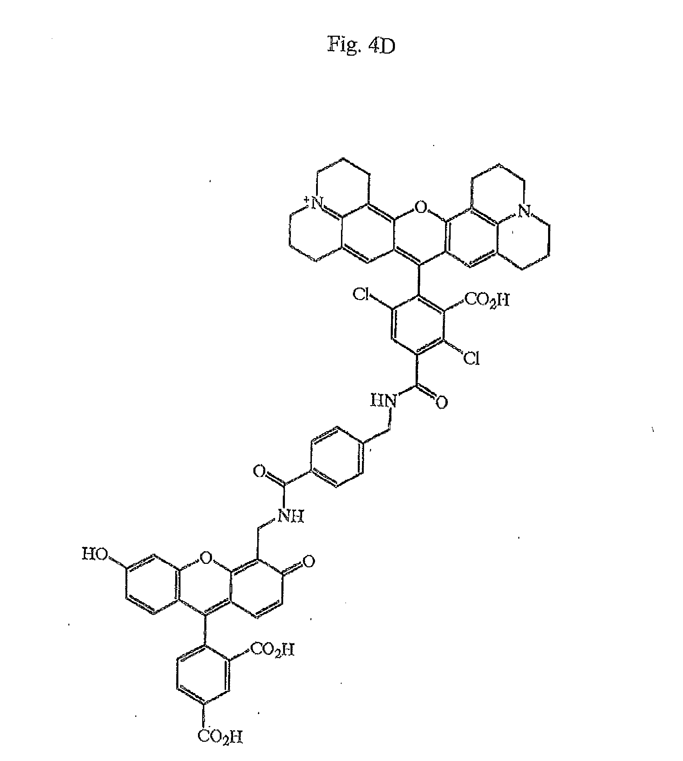

Fig. 3 shows the normalized fluorescence emission spectra of oligonucleotides respectively including the four energy transfer dyes shown in Figs. 4A-4D. The energy transfer dye fluorescence values shown in Fig. 3 include the values, represented as peaks shown respectively from left to right, for 5-CFB-DR110-2 (Fig. 4A), 5-CFB-DR6G-2 (Fig. 4B), 6-CFB-DTMR-2 (Fig. 4C), and 6-CFB-DROX-2 (Fig. 4D). More details about these energy transfer dyes can be found in U.S. Patent No. 5,945,526 to Lee et al., which is incorporated herein in its entirety by reference. As can be seen from Fig. 3, all four energy transfer dyes have individual peak fluorescence emission ranges that are spectrally different from the peak fluorescence ranges of the other energy transfer dyes. According to various embodiments, the four dyes would not be used together in a set of dyes. Instead, any one of the energy transfer dyes shown in Figs. 4A-4D could be used with one or more other dyes having the same, or having a substantially overlapping, emission wavelength range.

EXAMPLE 2 - The following example compares the absorption and emission frequencies of donor dyes that can be used as the donor dye moieties of three respective energy transfer dyes that can be used together in a set according to some embodiments. Emission spectra from the various donor dyes were obtained at a primer concentration of 0.4 µM in 8M urea, 1 X Tris/Borate/EDTA (TBE) buffer, using three excitation sources emitting at 500 nm, 560 nm, and 590 mn, respectively.

The absorption and emission characteristics of the three donor dyes are presented in Table 2. As shown in Table 2, the absorption properties of the three donor dyes are spectrally resolvable from one another. A set of energy transfer dyes that comprise respective moieties of these three donor dyes provide dyes having spectrally different and resolvable absorption characteristics. A set of dyes according to some embodiments can comprise at least one'dye formed from a moiety of any one of the donor dyes listed in Table 2 and conjugated or linked to an acceptor dye moiety made from a first dye. The set can also comprise the first dye and/or at least one other energy transfer dye that also includes an acceptor dye moiety made from the first dye.

**TABLE 2**

| **Dye** | **Absorption (nm)** | **Emission (nm)** |
|---|---|---|
| FAM | 492 | 515 |
| Rhodamine Red-X | 560 | 580 |
| ROX | 590 | 610 |

In some embodiments, at least two of the illustrated donor dyes listed in Table 2 can each individually be formed into a moiety and linked, for example, via a respective linker moiety or via a respective aminohexyl linkage, to a respective acceptor dye moiety. The acceptor dye moiety for each of the at least two illustrated dyes can be the same as or different than the acceptor dye moiety linked to the other of the at least two illustrated donor dyes. In an exemplary set, each of the FAM and Rhodamine Red-X donor dye moieties can be linked with a linker moiety to a respective ROX acceptor dye moiety. The set can also include ROX dye alone (without attachment to a donor dye moiety). The dyes in the exemplified set of dyes would have spectrally different respective peak absorption wavelength ranges, and the same peak emission wavelength range, that is, a peak emission wavelength range centered at about 610 nm. A monochromatic detector could be used in conjunction with the set to detect emission at 610 nm. Linking the FAM and Rhodamine Red-X dyes to respective ROX acceptor dye moieties can be accomplished by any of a number of linker moieties. The linker moieties can include, for example, aminohexyl linkers or the linkers described in U.S. Patents Nos. 5,800,996, 5,863,727, and 5,945,526, all to Lee et al, which are incorporated herein in their entireties by reference.

Those skilled in the art can appreciate from the foregoing description that the present teachings can be implemented in a variety of forms. Therefore, while these teachings have been described in connection with particular embodiments and examples thereof, the present teachings should not be so limited. Various changes and modifications can be made without departing from the present teachings. The following section of the description consists of numbered paragraphs simply providing statements of the invention already described herein. The numbered paragraphs in this section are not claims. The claims are set forth below in the later section headed "claims".
1. A system comprising:
   at least two excitation sources, each excitation source adapted to provide a different excitation wavelength range than at least one other of the excitation sources;
   at least one detector; and
   a plurality of dyes, wherein each dye of the plurality of dyes absorbs radiation at a substantially non-overlapping peak absorption range compared to the other dyes of the plurality, each dye of the plurality emits radiation within a peak emission wavelength range that substantially overlaps a peak emission wavelength range of at least one other dye of the plurality, at least a first dye of the plurality of dyes has a first peak absorption wavelength range that overlaps with the excitation wavelength range of a first of the at least two excitation sources, and at least a second dye of the plurality of dyes has a second peak absorption wavelength range that overlaps with the excitation wavelength range of a second of the at least two excitation sources.
2. The system of 1, wherein the plurality of dyes comprises at least one energy transfer dye comprising:
   a donor dye moiety excitable at a different excitation wavelength than at least one other dye of the plurality of dyes; and
   an acceptor dye moiety that emits radiation upon excitation at a same wavelength as a wavelength emitted by at least one other dye of the plurality of dyes, upon excitation.
3. The system of 1, wherein the plurality of dyes comprises at least two energy transfer dyes, wherein each energy transfer dye comprises:
   a donor dye moiety excitable at a different excitation wavelength than at least one other donor dye moiety of at least one of the other at least two energy transfer dyes; and
   an acceptor dye moiety that emits radiation upon excitation at a same wavelength as at least one other acceptor dye moiety of at least one of the other at least two energy transfer dyes, upon excitation.
4. The system of 3, wherein the donor dye moiety of at least one of the at least two energy transfer dyes comprises at least one of a ROX moiety and a TAMRA moiety.
5. The system of 1, wherein the system comprises a single high pass filter adjacent the at least one detector.
6. The system of 1, further comprising a controller for independently actuating the at least two excitation sources.
7. The system of 1, wherein the at least two excitation sources comprises one or more of a light-emitting diode, a solid-state laser, a quantum dot-based radiation-emitting diode, a nanotube field emitter radiation source, an organic LED, and a combination thereof.
8. The system of 1, wherein the at least one detector comprises one or more of a single wavelength detector, a multiple wavelength detector, a multiplex detector, a photodiode, a photodiode array, a charge-coupled device, a complementary metal oxide semiconductor, a photomultiplexer tube, an avalanche photodiode, and a combination thereof
9. The system of 1, wherein the first peak absorption wavelength range is 20 nanometers (nm) wide, the second peak absorption wave length range is 20 nm wide, and the first peak absorption wave length range overlaps with the second peak absorption wavelength range by no more than about ten percent.
10. The system of 1, wherein the first peak absorption wavelength range is 20 nanometers (nm) wide, the second peak absorption wave length range is 20 nm wide, and the first peak absorption wave length range overlaps with the second peak absorption wavelength range by no more than about two percent.
11. The system of 1, wherein the peak emission wavelength range of at least one dye of the plurality of dyes is a first range that is 20 nm wide, the peak emission wavelength range of at least one other dye of the plurality of dyes is a second range that is 20 nm wide, and the first range overlaps the second range by at least about fifty percent.
12. The system of 1, wherein the peak emission wavelength range of at least one dye of the plurality of dyes is a first range that is 20 nm wide, the peak emission wavelength range of at least one other dye of the plurality of dyes is a second range that is 20 nm wide, and the first range overlaps the second range by at least about seventy percent.
13. The system of 1, wherein at least one dye of the plurality of dyes is an energy transfer dye, and the energy transfer dye comprises a quencher dye moiety linked to at least one of a donor dye moiety, an acceptor dye moiety, and a linker moiety.
14. A kit comprising a plurality of fluorescent dyes, wherein each dye of the plurality of fluorescent dyes absorbs radiation within a substantially non-overlapping peak absorption wavelength range compared to the other dyes of the plurality, and each dye of the plurality emits radiation within a peak emission wavelength range that substantially overlaps a peak emission wavelength range of at least one other dye of the plurality.
15. The kit of 14, wherein the plurality of fluorescent dyes comprises one or more energy transfer dyes, each of said one or more energy transfer dyes comprising:
   a donor dye moiety adapted to absorb radiation within a first peak absorption wavelength range and emit excitation energy; and
   an acceptor dye moiety adapted to absorb excitation energy emitted by the donor dye moiety and emit radiation within a first peak emission wavelength range.
16. The kit of 15, wherein at least one of the one or more energy transfer dyes further comprises a linker moiety that links the respective donor dye moiety to the respective acceptor dye moiety,
17. The kit of 14, further comprising a single container, and wherein the plurality of fluorescent dyes is disposed within the single container.
18. The kit of 14, wherein each dye of the plurality of fluorescent dyes is contained in a respective container separate from at least one other dyes of the plurality of fluorescent dyes.
19. The kit of 14, wherein the plurality of dyes comprises two or more energy transfer dyes, each of the two or more energy transfer dyes comprises a respective donor dye moiety that has a different peak absorption wavelength range than the donor dye moiety of at least one other of the two or more energy transfer dyes, and each of the two or more energy transfer dyes comprises an acceptor dye moiety that has a respective peak emission wavelength range that overlaps with the peak emission wavelength range of the acceptor dye moiety of at least one other of the two or more energy transfer dyes.
20. The kit of 14, wherein each dye of the plurality of fluorescent dyes absorbs radiation at a respective peak absorption wavelength range of 20 nm, and each respective peak absorption wavelength range of 20 nm overlaps each of the other respective peak absorption wavelength ranges by an amount in the range of from zero percent to about ten percent.
21. The kit of 14, wherein each dye of the plurality of fluorescent dyes absorbs radiation at a respective peak absorption wavelength range of 20 nm, and each respective peak absorption wavelength range of 20 nm overlaps each of the other respective peak absorption wavelength ranges by an amount in the range of from zero percent to about two percent.
22. The kit of 14, wherein each respective peak absorption wavelength range comprises a range of 20 nm and is free of any overlap with any of the other respective peak absorption wavelength ranges.
23. The kit of 14, wherein each dye of the plurality of fluorescent dyes emits radiation, upon excitation, at a respective peak emission wavelength range of 20 nm, and each respective peak emission wavelength range of 20 nm overlaps, by at least about 50%, with at least one other peak emission wavelength range of 20 nm of at least one other dye of the plurality of fluorescent dyes.
24. The kit of 23, wherein each dye of the plurality of fluorescent dyes emits radiation, upon excitation, at a peak emission wavelength range of 20 nm that overlaps, by at least about 70%, with the peak emission wavelength range of 20 nm of at least one other dye of the plurality of fluorescent dyes.
25. The kit of 23, wherein each dye of the plurality of fluorescent dyes emits radiation, upon excitation, at a peak emission wavelength range of 20 nm that overlaps, by at least about 95%, with the peak emission wavelength range of 20 nm of at least one other dye of the plurality of fluorescent dyes.
26. The kit of 16, wherein at least one of the one or more energy transfer dyes further comprises a quencher dye moiety linked to at least one of the respective donor dye moiety, the respective acceptor dye moiety, and the respective linker moiety.
27. The kit of 14, wherein at least one of the plurality of fluorescent dyes is a non-(energy transfer) dye.
28. The kit of 14, wherein each dye of the plurality of fluorescent dyes comprises a labeled nucleotide or a labeled nucleic acid sequence.
29. The kit of 28, wherein the dyes of the plurality of fluorescent dyes are disposed together in a mixture.
30. A method comprising:
   providing a mixture comprising at least two dyes with a nucleic acid sequence-containing sample, wherein each dye of the plurality of dyes absorbs radiation within a substantially non-overlapping peak absorption wavelength range compared to the other dyes of the plurality, and each dye of the plurality emits radiation within a peak emission wavelength range that substantially overlaps a peak emission wavelength range of at least one other dye of the plurality;
   irradiating the sample with a first excitation wavelength range;
   detecting radiation emitted from the at least two dyes upon irradiation of the sample with the first excitation wavelength range;
   irradiating the sample with a second excitation wavelength range that differs from the first excitation wavelength range; and
   detecting radiation emitted from the at least two dyes upon irradiation of the sample with the second excitation wavelength range.
31. The method of 30, further comprising:
   independently actuating at least two excitation sources to provide the at least two different excitation wavelengths at two different respective times.
32. The method of 31, further comprising correlating the emitted radiation detected, with the independently actuated excitation sources.
33. The method of 31, wherein the independently actuating comprises independently actuating at least two light-emitting diodes.
34. The method of 30, further comprising:
   actuating a radiation source; and
   spectrally separating emission beams from the radiation source to form at least two excitation sources of at least two different respective excitation wavelength ranges.
35. The method of 34, wherein the spectrally separating comprises forming at least two excitation sources of at least two different respective excitation wavelength ranges, at two different respective times.
36. The method of 34, wherein the spectrally separating comprises forming at least two excitation sources of at least two different respective excitation wavelength ranges, at the same time.
37. The method of 34, wherein the spectrally separating comprises filtering emission beams from the radiation source.
38. The method of 30, wherein the plurality of dyes comprises at least one energy transfer dye, wherein each energy transfer dye comprises a respective donor dye moiety and a respective acceptor dye moiety, the donor dye moiety absorbs radiation at a different respective peak absorption wavelength range compared to a peak absorption wavelength range of at least one other dye of the at least two dyes, and the acceptor dye moiety emits radiation within a peak emission wavelength range that overlaps with a peak emission wavelength range of at least one other dye of the at least two dyes.
39. The method of 30, wherein the nucleic acid sequence-containing sample comprises at least one nucleic acid sequence that reacts with at least one of the dyes of the plurality of dyes.
40. The method of 30, wherein the nucleic acid sequence-containing sample comprises at least one DNA molecule that reacts with at least one of the dyes of the plurality of dyes.

## Claims

1. A kit comprising a plurality of fluorescent dyes, wherein each dye of the plurality of fluorescent dyes absorbs radiation within a substantially non-overlapping peak absorption wavelength range compared to the other dyes of the plurality, and each dye of the plurality emits radiation within a peak emission wavelength range that substantially overlaps a peak emission wavelength range of at least one other dye of the plurality.

2. A kit as claimed in claim 1, wherein each dye of the plurality of dyes is covalently attached to a polynucleotide probe which is covalently attached to at least one quencher moiety.

3. A kit as claimed in claim 1 or claim 2, wherein at least a first dye of the plurality of dyes has a first peak absorption wavelength range that overlaps with a first excitation wavelength range and at least a second dye of the plurality of dyes has a second peak absorption wavelength range that overlaps with a second excitation wavelength range and wherein the excitation wavelength ranges are different and non-overlapping.

4. A kit as claimed in claim 1, wherein the plurality of fluorescent dyes comprises one or more energy transfer dyes, each of said one or more energy transfer dyes comprising:
a donor dye moiety adapted to absorb radiation within a first peak absorption wavelength range and emit excitation energy; and
an acceptor dye moiety adapted to absorb excitation energy emitted by the donor dye moiety and emit radiation within a first peak emission wavelength range.

5. A kit as claimed in claim 4, wherein at least one of the one or more energy transfer dyes further comprises a linker moiety that links the respective donor dye moiety to the respective acceptor dye moiety.

6. A kit as claimed in claim 5, wherein at least one of the one or more energy transfer dyes further comprises a quencher dye moiety linked to at least one of the respective donor dye moiety, the respective acceptor dye moiety, and the respective linker moiety.

7. A kit as claimed in claim 1, wherein the plurality of dyes comprises two or more energy transfer dyes, each of the two or more energy transfer dyes comprises a respective donor dye moiety that has a different peak absorption wavelength range than the donor dye moiety of at least one other of the two or more energy transfer dyes, and each of the two or more energy transfer dyes comprises an acceptor dye moiety that has a respective peak emission wavelength range that overlaps with the peak emission wavelength range of the acceptor dye moiety of at least one other of the two or more energy transfer dyes.

8. A kit as claimed in claim 1, wherein each dye of the plurality of fluorescent dyes absorbs radiation at a respective peak absorption wavelength range of 20 nm, and each respective peak absorption wavelength range of 20 nm overlaps each of the other respective peak absorption wavelength ranges by an amount in the range of from zero percent to about ten percent, preferably by an amount in the range of from zero percent to about two percent.

9. A kit as claimed in claim 1, wherein each respective peak absorption wavelength range comprises a range of 20 nm and is free of any overlap with any of the other respective peak absorption wavelength ranges.

10. A kit as claimed in claim 1, wherein each dye of the plurality of fluorescent dyes emits radiation, upon excitation, at a respective peak emission wavelength range of 20 nm, and each respective peak emission wavelength range of 20 nm overlaps, by at least about 50%, with at least one other peak emission wavelength range of 20 nm of at least one other dye of the plurality of fluorescent dyes; optionally wherein each dye of the plurality of fluorescent dyes emits radiation, upon excitation, at a peak emission wavelength range of 20 nm that overlaps, by at least about 70% or at least about 95%, with the peak emission wavelength range of 20 nm of at least one other dye of the plurality of fluorescent dyes.

11. A kit as claimed in any preceding claim, wherein at least one of the plurality of fluorescent dyes is a non- (energy transfer) dye.

12. A kit as claimed in any of claims 1 to 10, wherein each dye of the plurality of fluorescent dyes comprises a labeled nucleotide or a labeled nucleic acid sequence.

13. A kit as claimed in claim 12, wherein the dyes of the plurality of fluorescent dyes are disposed together in a mixture.

14. A kit as claimed in any preceding claim, further comprising a single container, and wherein the plurality of fluorescent dyes is disposed within the single container.

15. A kit as claimed in any of claims 1 to 13, wherein each dye of the plurality of fluorescent dyes is contained in a respective container separate from at least one other dyes of the plurality of fluorescent dyes.
